# EUROPÄISCHE PATENTSCHRIFT

(11) **EP 0 519 872 B1**
(45) Veröffentlichungstag und Bekanntmachung des Hinweises auf die Patenterteilung: **17.01.1996**
(21) Anmeldenummer: 92810463.7
(22) Anmeldetag: 16.06.1992
(51) Int. Cl.: A61F 2/38

(54) **Gelenkprothese, insbesondere Kniegelenkprothese**
Joint prosthesis, in particular a knee prosthesis
Prothèse d'articulation, en particulier du genou

(30) Priorität: 17.06.1991 CH 1797/91; 17.06.1991 CH 1798/91
(43) Veröffentlichungstag der Anmeldung: 23.12.1992
(73) Patentinhaber: Bähler, André, CH-8032 Zürich (CH)
(72) Erfinder: Bähler, André, CH-8032 Zürich (CH)
(74) Vertreter: Riederer, Conrad A., Dr.

(56) Entgegenhaltungen:
- EP-A- 0 183 670
- FR-A- 2 290 883
- GB-A- 2 061 730
- US-A- 4 207 627

## Beschreibung

Die Erfindung betrifft eine Gelenkprothese, insbesondere Kniegelenkprothese, mit mindestens einem ersten Prothesenteil, das einen Verankerungsabschnitt und mindestens einen Gelenkabschnitt aufweist, mindestens einem zweiten Prothesenteil, das einen Verankerungsabschnitt und eine Platte aufweist, und mindestens einem Zwischenteil, welches mit dem Gelenkabschnitt des ersten Prothesenteils eine Artikulation ermöglicht und durch Zusammenwirken einer Führung mit einem Führungsorgan von anterior nach posterior und umgekehrt verschiebbar ist.

Ein bis heute nur unvollkommen gelöstes Problem der Endoprothetik ist die unveränderte Aufrechterhaltung eines stabilen Knochen/Implantat-Verbundes über lange Jahre, im Idealfall bis zum Tode des Prothesenträgers. Es sind vor allem zwei Faktoren, welche dagegen wirken. Es sind dies zum einen die an der Knochen/Implantat-Grenze, dem sogenannten Interface, auftretenden, nach Betrag und Richtung wechselnden Kräfte, insbesondere Scherkräfte, und zum andern knochenzerstörende, biologische Gewebereaktionen auf Fremdkörpermaterial, insbesondere auf Abriebpartikel von den Prothesenteilen. Soll also die Langlebigkeit einer Gelenkprothese erhöht werden, so sind durch geeignete Massnahmen die am Interface angreifenden Wechselkräfte klein zu halten und der an den Gleitflächen auftretende Verschleiss zu minimieren. Dies kann auf verschiedene Weise geschehen, doch sind nicht alle Methoden gleichermassen gangbar. Es genügt nämlich nicht, nur die genannten technischen Aspekte zu berücksichtigen, sondern es muss auch auf die anatomischen und physiologischen Verhältnisse Rücksicht genommen werden. Insbesondere bei Gelenken mit einer verhältnismässig komplizierten Kinematik, wie z.B. beim Kniegelenk, kann es zu schwer zu lösenden Zielkonflikten kommen.

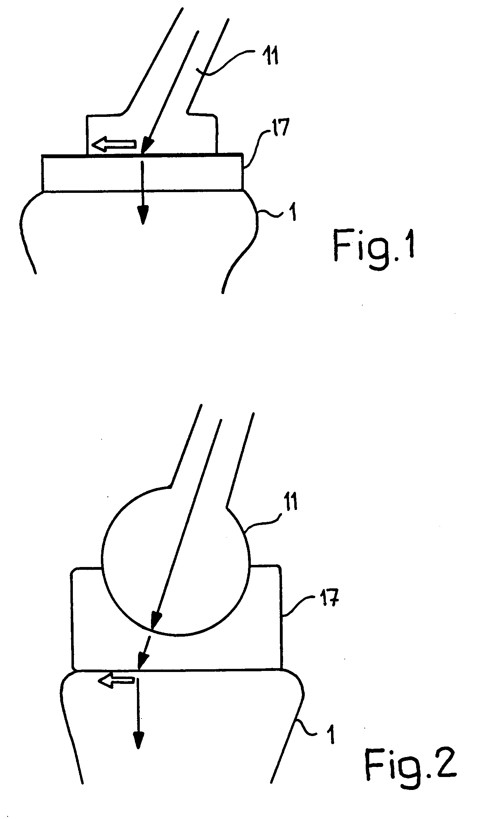

Bekanntlich ist bei Gleitlagern der Verschleiss umso geringer, je kleiner der Flächendruck der aufeinander reibenden Gleitpartner ist. Der Flächendruck, und damit auch der resultierende Verschleiss, ist klein, wenn die eigentliche Kontaktfläche der beiden Gleitpartner gross ist. Die Kontaktfläche ist dann gross, wenn die Gleitflächen möglichst gross und kongruent ausgebildet sind. Typische Beispiele hierfür sind das Schlittengelenk (Fig. 1) und das Scharniergelenk (Fig. 2). Obwohl das Schlittengelenk im Prinzip als ein Scharniergelenk mit unendlichem Radius betrachtet werden kann, bestehen aber ausser dem gemeinsamen Merkmal des minimierten Verschleisses grundsätzliche Unterschiede. Das Schlittengelenk ist translativ frei beweglich, besitzt aber keine Drehachse. Umgekehrt besitzt das Scharniergelenk eine Drehachse, ist aber translativ gefangen. Dies hat unterschiedliches Verhalten gegenüber von aussen einwirkenden Kräften zur Folge.

Beim Schlittengelenk bewirkt z.B. eine schräg von oben kommende Kraft eine Verschiebung des Gelenkkopfes auf der Gleitfläche in Richtung der horizontalen Komponente, ohne dass der untere Gelenkteil und damit dessen Interface von dieser Horizontalkraft selbst betroffen würde.

Beim Scharniergelenk hingegen geht die gleiche Kraft durch das Gelenk hindurch, ohne im Gelenk eine Bewegung auszulösen, induziert jedoch am Interface des unteren Gelenkteils eine Horizontalkomponente und damit eine unerwünschte Scherkraft, die zudem ständig ihre Richtung wechselt, wenn der obere Gelenkteil hin und her pendelt. Vereinfacht gesagt, wird mit dem Scharniergelenk der Vorteil der Drehbeweglichkeit mit dem Nachteil der translativen Immobilität und der Scherkraftbelastung am Interface erkauft. Nun sind Körpergelenke selten reine Scharnier- oder Schlittengelenke, vielmehr findet sich, wie z.B. beim Kniegelenk, eine Kombination davon, indem der Drehbewegung eine gleichzeitige Translation überlagert ist.

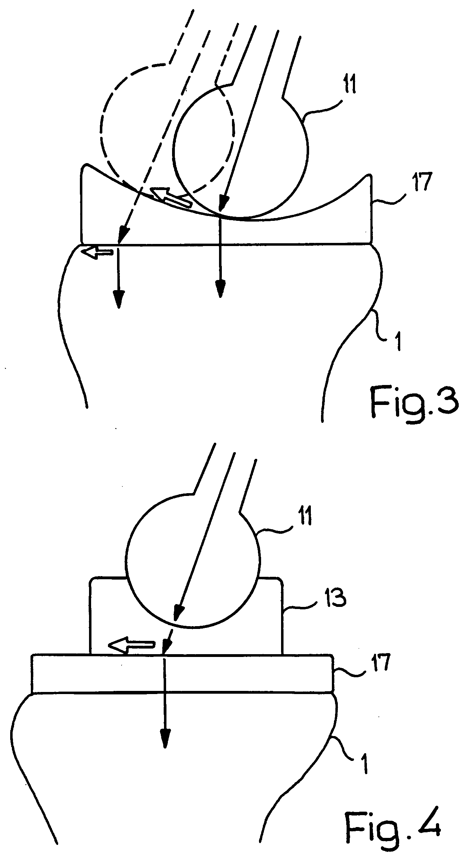

Um diese Kombination von Bewegungen zu ermöglichen, muss das Scharniergelenk "geöffnet" werden, d.h. die Kongruenz der Gleitflächen muss herabgesetzt werden (Fig. 3). Dies bedeutet aber kleine Kontaktflächen mit entsprechend grossem Flächendruck, was erhöhten Verschleiss zur Folge hat. Der Verschleiss wird zusätzlich durch die repetitive Translation des Gelenkkopfes auf der Gleitfläche erhöht, was infolge des fokussierten Flächendrucks zu einem Walkprozess mit besonders schneller Materialermüdung des untenliegenden Gelenkteils führt. Zudem schützt die Inkongruenz nicht unbedingt vor Scherkräften am Interface. Ueberhöhte Gleitflächen an der Peripherie wirken sich kräftemässig analog aus, wie ein Scharniergelenk. Diese Nachteile, erhöhter Verschleiss und Scherkräfte am Interface mit den damit verbundenen negativen Auswirkungen, sind bei allen Prothesen dieser Bauart wohlbekannt.

Eine andere bekannte Lösung ist die Kombination von Schlitten- und Scharniergelenk auf zwei Ebenen mit Hilfe eines Zwischenteils (Fig. 4). Der Drehbewegung des Scharniergelenks wird gewissermassen die Translationsbewegung des Schlittengelenks unterlagert. Bei diesem Prinzip bleibt die Kongruenz der Gelenkflächen voll erhalten, und der Verschleiss wird minimiert. Zudem werden die durch das Scharniergelenk durchgehenden Kräfte nicht auf das Interface übertragen, sondern im Zwischenteil in Translationsbewegung umgesetzt. Es existieren auch Prothesen dieser Bauart und sind als sogenannte Meniskus-Knie bekannt.

So werden in der EP-B-0 021 421 zwei verschiedene Kniegelenkprothesen beschrieben, welche auch als "Oxford-Knie" und "New Jersey-Knie" bezeichnet werden. Das Oxford-Knie weist zwei Femurteile, zwei Zwischenteile und zwei Tibiateile auf. Die Femurteile bestehen aus je einem sphärischen Segment, das einen Verankerungsabschnitt zur Verankerung im Femur aufweist. Die Zwischenteile sind runde Scheiben mit einer sphärischen Vertiefung zur Lagerung des entsprechenden Femurteils. Die Tibiateile besitzen einen Verankerungsabschnitt zur Verankerung in der Tibia und ein Plateau, auf welchem das Zwischenteil gleiten kann. Als nachteilig erweist sich dabei, dass bei einer Flexion von 90 Grad und mehr das Zwischenteil über das Plateau teilweise hinausgeschoben und eventuell ganz disloziert werden kann. Die gleiche Gefahr besteht auch, wenn aus irgendwelchen Gründen die Spannung der nach der Operation verbliebenen Gelenkbänder nachlässt und sich somit die Femurteile von den Zwischenteilen abheben lassen. In solchen Fällen ist stets eine erneute Operation des Kniegelenks notwendig.

Das New Jersey-Knie begegnet der beschriebenen Gefahr des Hinausschiebens durch spezielle Vorrichtungen. Am Tibiateil, das für beide Zwischenteile vorgesehen ist, befinden sich zwei schwalbenschwanzartige, bogenförmige Nuten, durch welche die Zwischenteile zwangsgeführt werden. Die Bogen sind jeweils gegen das Zentrum zu gerichtet, so dass das Zwischenteil beim Beugen des Knies nicht unkontrolliert rückwärtsgleiten und über das Plateau hinausgeschoben werden kann, sondern am dort noch verbliebenen zentralen Knochenzapfen anschlägt. Die Schwalbenschwanzform andererseits verhindert ein Luxieren des Zwischenteils beim Nachlassen der Bänderspannung. Der Nachteil dieser Ausführung ist die Zwangsführung der Zwischenteile auf eine vorbestimmte Bahn, welche nur für eine einzige, singuläre Gelenkstellung eine Kongruenz der Gelenkflächen zwischen Femurteil und Zwischenteil zulässt, für jede andere Gelenkstellung jedoch zu einer verschleissfördernden Inkongruenz der Gelenkflächen führt. Eine Inkongruenz entsteht, weil die am Femur fest verankerten Femurteile stets im gleichen Abstand zueinander stehen und eine gemeinsame Drehachse haben, die Zwischenteile jedoch beim Vor- und Rückwärtsgleiten auf der bogenförmigen Bahn sich seitlich einander nähern oder voneinander entfernen, wobei ausserdem die Scharniergelenkachsen stetig ihre Stellung zueinander ändern. Dasselbe, nur im umgekehrten Sinne, geschieht auch bei Rotationsbewegungen um eine Achse senkrecht zum Plateau. Der ständig wechselnde Grad der dieser Prothese innewohnenden Inkongruenz der Scharniergelenkflächen kompromittiert natürlich das verschleissminimierende Prinzip des Meniskusknie in grundsätzlicher Weise.

In der DE-A-30 39 992, zu deren Patentfamilie auch die GB-A-2061730 gehört, wird eine Kniegelenkprothese beschrieben, welche ein Femurteil, ein Tibiateil und ein Zwischenteil aufweist. Das Femurteil besitzt einen Verankerungsabschnitt zur Befestigung im Femur und zwei Kondylen. Das Tibiateil besitzt einen Verankerungsabschnitt zur Befestigung in der Tibia und eine abgeflachte Lagerfläche mit einer sich darin erstreckenden gebogenen Nut zur Aufnahme einer Rippe des Zwischenteils. Das Zwischenteil besitzt eine in die gebogene Nut des Tibiateils passende Rippe sowie zwei konkave Gleitlager zur Lagerung der Kondylen des Femurteils. Dadurch weist diese Prothese den Vorteil der verschleissmindernden Kongruenz der Gleitflächen des Scharniergelenks auf.

Im Gegensatz jedoch zu den vorhergehend diskutierten Beispielen besteht wegen der gebogenen Nut und Rippe keine Möglichkeit der translativen Bewegung des Femurteils relativ zum Tibiateil, sondern nur die Möglichkeit der Rotation um die Achse der Bogennut. Es handelt sich hier also um ein klassisches Scharniergelenk mit der zusätzlichen Freiheit der Rotation um eine senkrecht zu diesem Gelenk stehende Achse. Demzufolge gehen auch schräg von oben kommende Kräfte, wie sie z.B. durch den Muskelzug oder die Gewichtsbelastung beim Aufsetzen des Fusses entstehen, durch das Gelenk hindurch auf das Interface und induzieren dort die unerwünschten Scherkräfte. Auch in diesem Fall wird das Prinzip des Meniskusknie kompromittiert, indem die verschleissmindernde Kongruenz der Gelenkflächen mit dem Verlust der verankerungsschonenden Translationsmöglichkeit und der damit verbundenen natürlichen Kniekinematik erkauft wird. Ausserdem ist wegen der gebogenen Nut die Verwendung luxationsverhindernder Mittel, wie z.B. eine Schwalbenschwanzform, nicht ohne weiteres möglich, vor allem dann nicht, wenn bei bereits implantiertem Tibiateil das Zwischenteil nachträglich eingesetzt werden soll. Ueber die sich daraus ergebenden Nachteile wurde bereits beim "Oxford"-Knie diskutiert.

Eine Kniegelenkprothese mit einem Femurteil, Tibiateil und Zwischenteil wird auch in der GB-B-1 567 007 beschrieben. Das Femurteil besitzt einen Verankerungsabschnitt zur Befestigung im Femur sowie eine Kondyle. Das Tibiateil besitzt einen Verankerungsabschnitt zur Befestigung in der Tibia sowie eine gerade, schwalbenschwanzförmige Nut zur Aufnahme einer entsprechenden Rippe des Zwischenteils. Das Zwischenteil besitzt eine gerade, in die Nut passende schwalbenschwanzförmige Rippe sowie ein pfannenförmiges Gleitlager zur Artikulation mit der Kondyle des Femurteils. Auch diese Prothese weist den Vorteil der verschleissmindernden Kongruenz der Gleitflächen auf, und zudem wird durch die Schwalbenschwanzführung eine Luxation des Zwischenteils im Sinne eines unerwünschten Abhebens vom Tibiateil verhindert.

Der Nachteil dieser Ausführung besteht darin, dass zwar eine Translationsbewegung in der Längsrichtung der Nut möglich ist, aber keine Rotationsbewegung um eine Achse senkrecht zum Tibiaplateau. Da jedoch solche Rotationsbewegungen praktisch jeder Bewegung des Kniegelenks überlagert sind, kommt es zu einer andauernden Inkongruenz der Gleitflächen von Femur- und Zwischenteil und somit zu einer Behinderung der natürlichen Kinematik des Kniegelenks.

Es ist daher Aufgabe der vorliegenden Erfindung, die beschriebenen Nachteile bekannter Prothesen zu vermeiden. Insbesondere soll eine Prothese geschaffen werden, welche nicht nur Gelenkflächenkongruenz und Translationsmöglichkeit des sogenannten Meniskusknies aufweist, sondern auch weitgehende Luxationssicherheit des Zwischenteils gewährleistet und einen mindestens annähernd normalen physiologischen Bewegungsablauf ermöglicht.

Diese Aufgabe wird gemäss der Erfindung bei einer Gelenkprothese der eingangs erwähnten Gattung dadurch gelöst, dass die Führung gegen anterior und posterior aufgeweitet ist, um dem Zwischenteil eine entsprechende Bewegungsfreiheit zu gestatten.

Die erfindungsgemässe Prothese ermöglicht einen Bewegungsablauf, der weitgehend dem normalen physiologischen Bewegungsablauf entspricht. So verlagern sich bei der Flexion die Kondylen von ventral nach dorsal. Gleichzeitig kann aber auch eine Drehbewegung stattfinden. Sowohl bei der Flexion als auch bei der Extension sind daher ähnliche Verhältnisse gegeben wie beim natürlichen Knie. Auch ist in jeder Gelenkstellung die Kongruenz der Scharniergelenkflächen von Femurteil und Zwischenteil gewährleistet, da die Zwischenteile nicht starr geführt sind, sondern sich in den Nuten mit seitlichem Spiel bewegen können, so dass die auf den Zwischenteilen angeordneten Gelenkflächen zueinander ausgerichtet bleiben. Von oben kommende Kräfte gelangen nicht zum Interface, sondern werden im Zwischenteil in Translationsbewegung umgesetzt. Schliesslich ist auch die Gefahr der gefürchteten Dislokation des Zwischenteils bei der Flexion praktisch gebannt, weil der Bewegungsablauf weitgehend dem normalen physiologischen Bewegungsablauf entspricht.

Da das Zwischenteil ein relativ kleines auswechselbares Element ist, ist es möglich, intraoperativ verschieden hohe Zwischenteile einzupassen, um einseitige Bandlaxizitäten auszugleichen oder Achsfehlstellungen zu korrigieren. Es ist auch möglich, Zwischenteil und ersten und zweiten Prothesenteil aus verschiedenen Materialien zu fertigen. So könnten beispielsweise das Zwischenteil aus Kunststoff und die Prothesenteile aus Metall bestehen.

Die jeweilige Führung wird zweckmässigerweise durch eine Seitenwände aufweisende Nut in der Platte gebildet. Dies ergibt eine einfache Konstruktion. Um die Führung, d.h. die Nut, gegen anterior und posterior erfindungsgemäss ausgeweitet zu gestalten, sind verschiedene Formgebungen der Seitenwände der Nut möglich. Vorteilhaft weisen die Seitenwände der Nut mindestens gegen den Rand der Platte hin eine Krümmung auf, so dass die Nut in einem Bogen um die zentrale Achse verläuft. Auch das Führungsorgan weist eine entsprechende länglich gekrümmte Form auf. Dies ermöglicht einen Bewegungsablauf bei dem eine Rotation bei der Flexion und Extension in ähnlicher Weise erfolgen kann wie beim natürlichen Gelenk. Zweckmässigerweise ist der Abstand zwischen den Seitenwänden der Nut etwa in der Mitte der Nutlänge am kleinsten, wobei die Breite des Führungsorgans über dessen Länge nicht ganz diesem Abstand entspricht. Dadurch ergibt sich ein seitliches Spiel, das in anterior/posteriorer Richtung eine Translationsmöglichkeit des Zwischenteils relativ zum zweiten Prothesenteil erlaubt. Die freie Wegstrecke der Translation hängt dabei im wesentlichen von der halben Breitendifferenz von Nut und Führungsorgan ab sowie von den Radien der konkaven Seitenwände derselben. Sie ist mit beiden positiv korreliert. Die freie Wegstrecke nimmt also bei grösserer Differenz in der Breite und/oder grösseren Radien zu. So beträgt beispielsweise die Breitendifferenz bei einer angenommenen Translationsstrecke von 15 mm und einem Radius der äusseren Seitenwand der Nut von 50 mm, wie sie für ein Kniegelenk als angemessen zu betrachten sind, 1.13 mm, bei einem freien Translationsweg von 25 mm und einem Radius 70 mm, die als eher grosszügig einzustufen sind, 2.25 mm. In beiden Fällen bewegt sich das seitliche Spiel noch innerhalb der Toleranz-Grenzen, die für ein stabiles Knie als normal angesehen werden.

Eine besonders vorteilhafte Ausführungsform der Erfindung sieht vor, dass die innere Seitenwand der Nut gekrümmt ist, dass die äussere Seitenwand der Nut zumindest in der Mitte einen praktisch geraden Abschnitt aufweist und dass umgekehrt die äussere Seitenwand des Führungsorgans gekrümmt ist und die innere Seitenwand des Führungsorgans zumindest in der Mitte einen geraden Abschnitt aufweist. Dies ermöglicht es, das seitliche Spiel zwischen den Führungsorganen der Zwischenstücke und den Führungsbahnen besonders klein zu halten, so dass eine hohe Querstabilität des Gelenks erzielt wird, was insbesondere für Kniegelenkprothesen wichtig ist. Die beschriebene Formgebung schafft aber in anterior/posteriorer Richtung eine Translationsmöglichkeit der Zwischenteile relativ zum zweiten Prothesenteil.

Der Radius der Krümmung der äusseren Seitenwand des Führungsorgans ist gleich gross oder kleiner als der Radius eines konzentrischen Kreises um die innere Seitenwand der Führung, welcher den geraden Abschnitt der äusseren Seitenwand der Führungsbahn berührt. Der Radius des konzentrischen Kreises ist etwa 1,3 bis 2,6 mal grösser als der Radius der Krümmung der inneren Seitenwand der Führung. Durch diese Dimensionierung wird eine anteriore und posteriore Ausweitung der Führung erreicht, die die gewünschte Bewegungsfreiheit für den Zwischenteil bei seiner Bewegung nach anterior oder posterior schafft. Die Verwendung von Kreiskurven hat herstellungstechnische Vorteile. Es könnten auch andere Kurven, z.B. elliptische, Anwendung finden. Die Erfindung erstreckt sich daher auch auf äquivalente Formgebungen.

Das seitliche Spiel des Führungsorgans in der Führung an deren engster Stelle liegt vorteilhaft im Bereich von 0,5 bis 3 mm. Eine zweckmässige Ausführungsform sieht vor, dass der Radius der Krümmung der äusseren Seitenwand der Nut etwa dreimal grösser ist als der Radius der inneren Seitenwand. Durch diese Dimensionierung wird eine anteriore und posteriore Ausweitung der Führungsbahn erreicht, die die gewünschte Bewegungsfreiheit für den Zwischenteil bei seiner Bewegung nach anterior oder posterior schafft. Der Radius der Krümmung der äusseren Seitenwand des Führungsorgans ist zweckmässigerweise etwa halb so gross wie der Radius der Krümmung der äusseren Seitenwand der Nut. Die Verwendung von Kreiskurven hat herstellungstechnische Vorteile. Es könnten auch andere Kurven, z.B. elliptische, Anwendung finden. Die Erfindung erstreckt sich daher auch auf äquivalente Formgebungen.

Die Konstruktion ist vorzugsweise so gestaltet, dass die Beweglichkeit des Zwischenteils relativ zur Führung auf Bewegungen in der Gleitbahnebene eingeschränkt ist. So ist es möglich, der Führung einen schwalbenschwanz-, T-förmigen, polygonalen oder kreisförmigen Querschnitt zu geben. Durch diese Ausgestaltung wird verhindert, dass das Führungsorgan die Führung verlässt, wenn aus irgendwelchen Gründen die Spannung der nach der Operation verbliebenen Gelenkbänder nachlässt.

Die Drehgelenkabschnitte können verschiedenartig ausgebildet sein, um eine Drehbewegung zu ermöglichen. Eine vorteilhafte Ausführungsform sieht zwei Kondylen und für jede Kondyle ein Zwischenteil mit einer entsprechenden Lagerfläche vor. Die Führungen sind dabei vorteilhaft so angeordnet, dass sie anterior und posterior konvergieren. Dies ergibt eine Prothese, die dem natürlichen Gelenkaufbau, wie er beispielsweise beim Kniegelenk zu finden ist, sehr nahe kommt.

In einer Variante dieser Ausführungsform werden die beiden Zwischenteile nachträglich, nachdem sie in die Führungsnut eingelegt worden sind, mit einem brillen- oder brückenförmigen oder sonstwie geeignet geformten Verbindungsteil zusammengekoppelt. Dies verhindert eine relative Verschieblichkeit der Zwischenteile zueinander und damit eine unbeabsichtigte Inkongruenz der Drehgelenkflächen. Weiter bewirkt sie eine erhöhte Luxationssicherheit, indem die beiden zusammengekoppelten Zwischenteile innerhalb der gebogenen Nut gefangen werden und nicht mehr nach anterior oder posterior aus dieser austreten können. Zum dritten bewirkt sie eine gedämpfte Bremsung der Translationsbewegung in den Endpunkten der Bewegung. Die Bremsung ist besonders weich, wenn durch eine entsprechende Wahl der Radien dafür gesorgt wird, dass zuerst die konkaven Innenflächen der Führungsorgane an der konvexen Seitenwand der Nut auflaufen. Die Brillenform des Verbindungsteils ihrerseits hat den Vorteil, dass bei Benutzung von Kunststoff als Material für die Zwischenteile der Kaltfluss und damit ein weiterer Faktor für vorzeitige Materialermüdung und -verschleiss ausgeschaltet wird.

Möglich ist auch eine monokompartimentäre Ausführung der Gelenkprothese. So kann der Drehgelenkabschnitt des ersten Prothesenteils durch eine einzige Kondyle gebildet sein und für diese Kondyle eine Lagerfläche auf einem einzigen Zwischenteil vorgesehen sein.

Vorteilhafterweise ist die jeweilige Kondyle des ersten Prothesenteils konvex und die zugehörige Lagerfläche des Zwischenteils entsprechend konkav. Dies entspricht dem natürlichen Gelenkaufbau. Es wäre aber auch möglich, z.B. wo die anatomische Struktur dies nahelegt, die jeweilige Kondyle des ersten Prothesenteils als konkave Lagerfläche und die entsprechende Lagerfläche des Zwischenteils als konvexe Kondyle zu gestalten.

Bei der Ausbildung der Prothese als Kniegelenkprothese ist es vorteilhaft, wenn das zweite Prothesenteil eine Ausnehmung für das vordere und/oder hintere Kreuzband aufweist. Die erfindungsgemässe Ausbildung der Prothese lässt eine solche Ausnehmung zu. Dies hat den Vorteil, dass die für den Bewegungsablauf des Kniegelenks wichtige Funktion der Kreuzbänder aufrechterhalten wird.

Ausführungsbeispiele der Erfindung werden nun unter Bezugnahme auf die Zeichnung beschrieben. Es zeigt:
- Figur 1: die Kraftumsetzung in Translationsbewegung bei einem Schlittengelenk,
- Figur 2: die Scherkraftbildung am Interface bei einem Scharniergelenk,
- Figur 3: die Verhältnisse bei einem "geöffneten" Scharniergelenk,
- Figur 4: die Verhältnisse bei einem kombinierten Schlitten- und Scharniergelenk,
- Figur 5: ein erstes Ausführungsbeispiel einer Kniegelenkprothese im Schnitt entlang der Linie V-V von Fig. 6, wobei von den Zwischenteilen die Führungsorgane und gestrichelt die Umrisse dargestellt sind,
- Figur 5A: eine erste Variante zum Ausführungsbeispiel von Fig. 5 mit einem die beiden Zwischenteile verbindenden brillenförmigen Verbindungsteil,
- Figur 5B: eine zweite Variante zum Ausführungsbeispiel von Fig. 5 mit einem die beiden Zwischenteile verbindenden klammerförmigen Verbindungsteil,
- Figur 6: einen Schnitt entlang der Linie VI-VI von Fig. 5,
- Figur 6A: einen Schnitt entlang der Linie VIA-VIA von Fig. 5A,
- Figur 6B: einen Schnitt entlang der Linie VIB-VIB von Fig. 5B,
- Figur 6C: eine Kniegelenkprothese wie in Figur 6A in Explosionsdarstellung, wobei aber das Führungsorgan und Führungsbahn schwalbenschwanzförmig sind,
- Figur 7: das zweite Prothesenteil von Figur 6, 6A oder 6B, ohne die Zwischenteile,
- Figur 8: ein Zwischenteil von unten gesehen,
- Figur 9: ein zweites Ausführungsbeispiel einer Kniegelenkprothese,
- Figur 10A: einen Schnitt entlang der Linie X-X von Fig. 9,
- Figur 10B: eine Kniegelenkprothese wie in Fig. 10A in Explosionsdarstellung, wobei aber Führungsbahn und Führungsorgan wie in Fig. 11 ausgebildet sind,
- Figur 11: eine andere Ausführung von Führungsbahn und Führungsorgan als in Fig. 10,
- Figur 12: eine Kniegelenkprothese wie in Figur 5, wobei jedoch die Führung im Zwischenteil und das Führungsorgan beim zweiten Prothesenteil ausgebildet ist,
- Figur 13A: einen Schnitt entlang der Linie XIII-XIII von Figur 12,
- Figur 13B: eine Kniegelenkprothese wie in Figur 13A in Explosionsdarstellung, wobei aber das Führungsorgan und Führungsbahn schwalbenschwanzförmig sind,
- Figur 14: ist Ausführungsbeispiel einer Kniegelenkprothese, welche ähnlich wie jene von Figur 5 ist, aber eine andere Ausgestaltung von Führungsbahn und Führungsorgan aufweist,
- Figur 15: einen Schnitt entlang der Linie XV-XV von Fig. 14,
- Figur 16: ist eine Ansicht von oben auf ein anderes Ausführungsbeispiel entsprechend dem Schnitt XVI-XVI von Figur 17,
- Figur 17: ist ein Schnitt entlang der Linie XVII-XVII von Figur 16,
- Figur 18: eine Ansicht von oben auf ein weiteres Ausführungsbeispiel,
- Figur 19: einen Schnitt entlang der Linie XIX-XIX von Fig. 18,
- Figur 20: ein Zwischenteil von unten gesehen,
- Figur 21: das zweite Prothesenteil ohne die Zwischenteile von oben gesehen,
- Figur 22: ein weiteres Ausführungsbeispiel einer Kniegelenkprothese,
- Figur 23: einen Schnitt entlang der Linie XXIII-XXIII von Fig. 22,
- Figur 24: eine andere Ausführung von Führungsbahn und Führungsorgan als in Fig. 23,
- Figur 25: eine Kniegelenkprothese wie in Figur 18, wobei jedoch die Führung im Zwischenteil und das Führungsorgan beim zweiten Prothesenteil ausgebildet ist,
- Figur 26: einen Schnitt entlang der Linie XXVI-XXVI von Figur 25.

Bei den bereits einleitend behandelten verschieden bekannten Gelenkformen der Figuren 1 bis 4 ist die Tibia mit der Bezugsziffer 1 bezeichnet. Auf der Tibia 1 ist das untere Gelenkteil 17 befestigt. Die Bezugsziffer 11 bezeichnet das obere Gelenkteil. In Figur 4 findet sich noch ein auf dem unteren Gelenkteil 17 verschiebbares Zwischenteil 13.

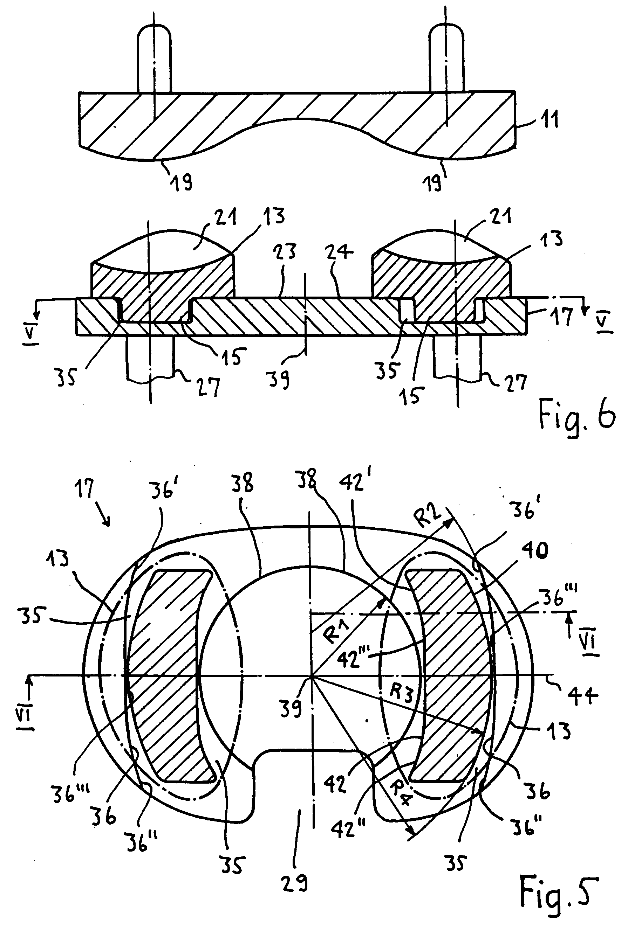

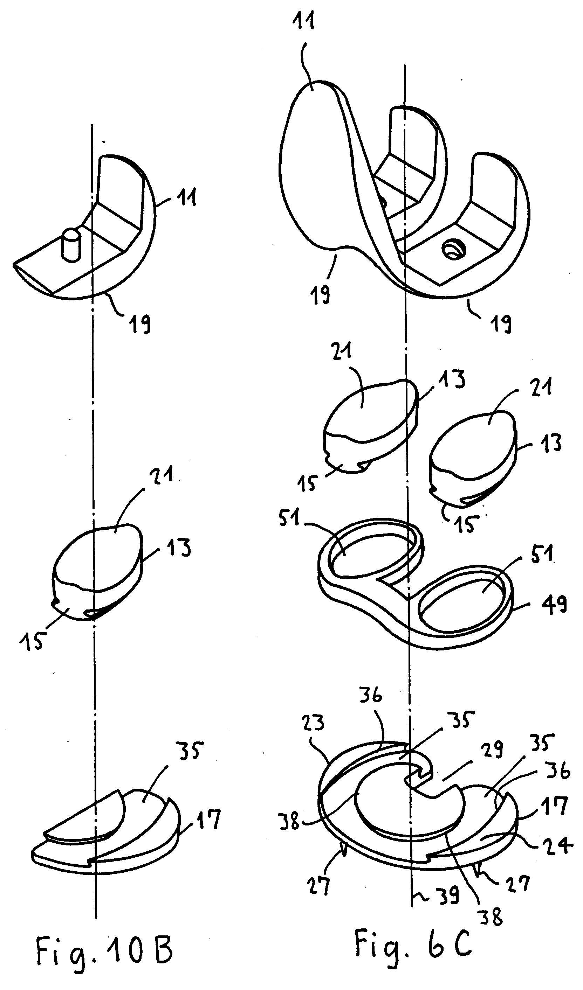

Für das Verständnis der in den Figuren 5 bis 8 dargestellten Kniegelenkprothesen betrachtet man am besten die Explosions-darstellung von Fig. 6C. Die Kniegelenkprothese besitzt ein erstes Prothesenteil 11, ein zweites Prothesenteil 17 und zwei Zwischenteile 13.

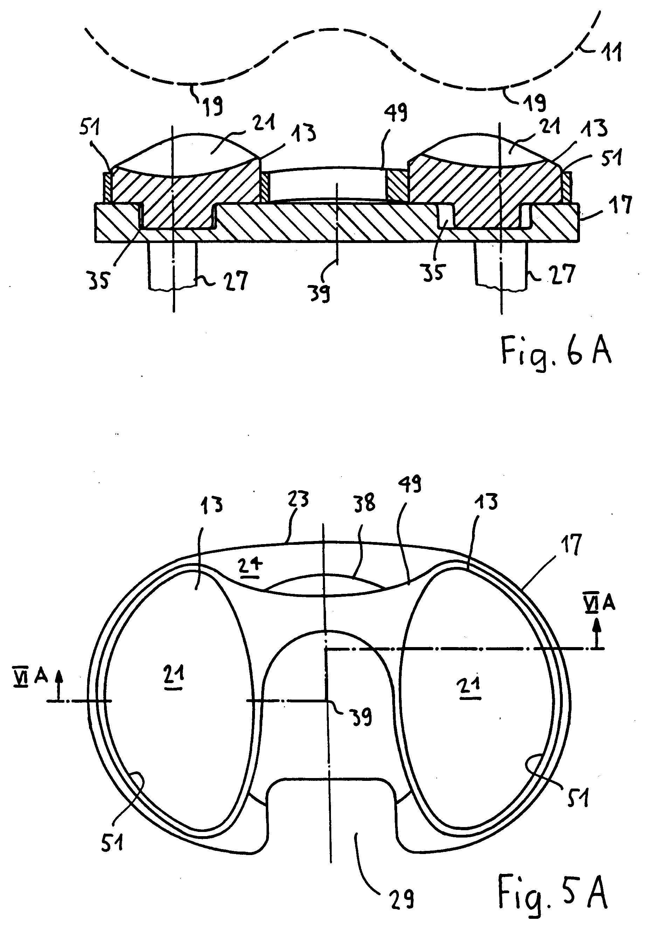

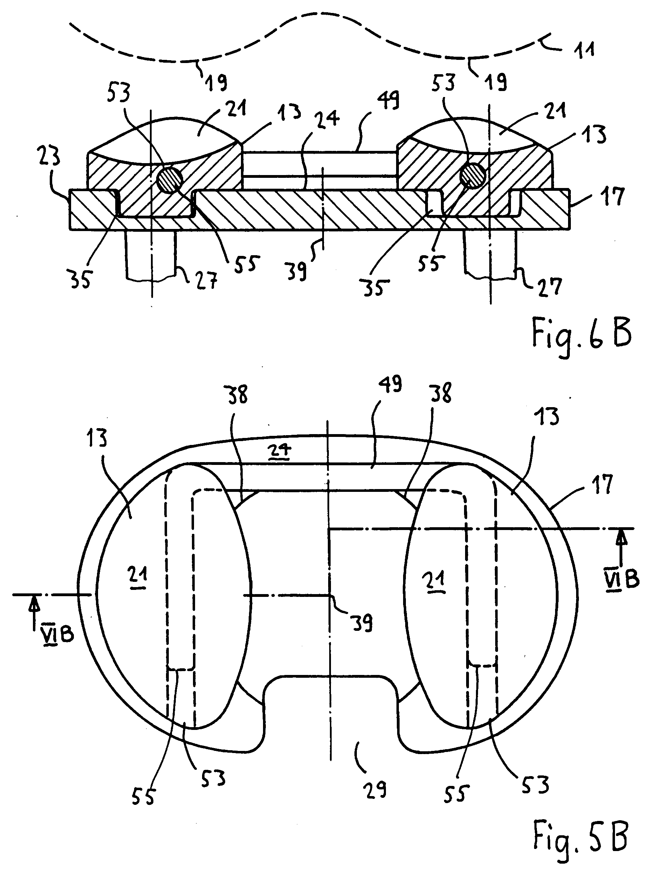

Das erste Prothesenteil 11 kann in herkömmlicher Weise mindestens einen Verankerungsabschnitt und mindestens einen Drehgelenkabschnitt aufweisen. Für die vorliegende Erfindung ist natürlich von Bedeutung, dass der Drehgelenkabschnitt des ersten Prothesenteils mit jenem des Zwischenteils übereinstimmt. Wie in den Figuren 6 und 6C dargestellt, wird der Drehgelenkabschnitt des ersten Prothesenteils 11 durch zwei Kondylen 19 gebildet. Diesen Kondylen 19 entsprechen die Lagerflächen 21 der Zwischenteile 13.

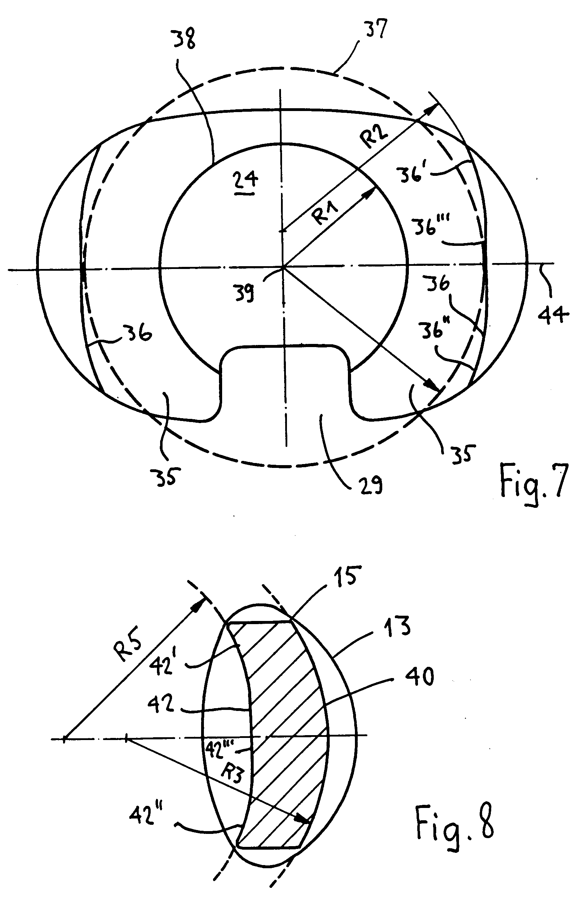

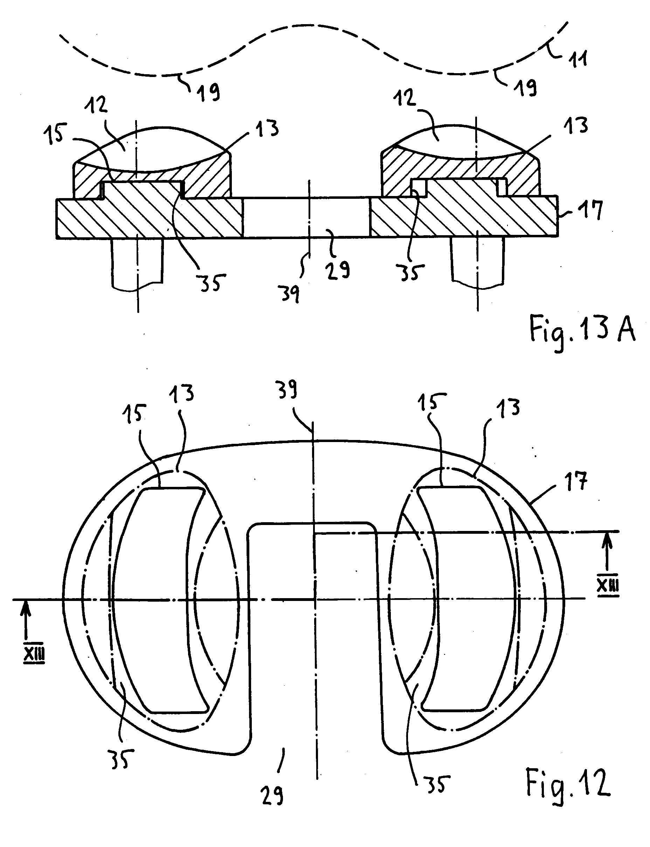

Das zweite Prothesenteil 17 besteht aus einer eine Gleitfläche 24 aufweisenden Platte 23, von welcher sich konische Verankerungsabschnitte 27 nach unten erstrecken. Wie die Figuren 5 und 7 zeigen, besitzt die Platte 23 eine den Umrissen der natürlichen Tibiakondylen angepasste ovale Formgebung mit einer posterioren Ausnehmung 29 für das hintere Kreuzband. Es ist jedoch möglich und ein Vorteil der vorliegenden Erfindung, dass die Ausnehmung 29 tief gestaltet werden kann, dass sie auch das vordere Kreuzband umfasst (Figur 12). Im Prothesenteil 17 befinden sich zwei Führungen 35, welche die Form einer gewöhnlichen Nut oder einer T- oder Schwalbenschwanz-Nut aufweisen. Sie könnten aber auch einen anderen geeigneten Querschnitt aufweisen. Jede Nut 35 ist gegen anterior oder posterior aufgeweitet. Die Seitenwände 38 sind gekrümmt. Die Seitenwände 36 weisen anterior und posterior eine Krümmung 36′, 36′′ auf, besitzen aber in der Mitte einen praktisch geraden Abschnitt 36′′′. Der Abstand zwischen den Seitenwänden 36, 38 ist etwa in der Mitte der Führung am kleinsten.

Die Formgebung der Zwischenteile 13 ist insbesondere aus den Figuren 5, 6 und 8 ersichtlich. Die Zwischenteile besitzen oben eine Lagerfläche 21 und unten ein in die Nut 35 ragendes Führungsorgan 15. Das Führungsorgan 15 hat eine längliche gekrümmte Form. Die Seitenwand 40 des Führungsorgans 15 ist gekrümmt. Die Seitenwand 42 weist anterior und posterior eine Krümmung 42′, 42′′ auf, besitzt aber in der Mitte einen praktisch geraden Abschnitt 42′′′.

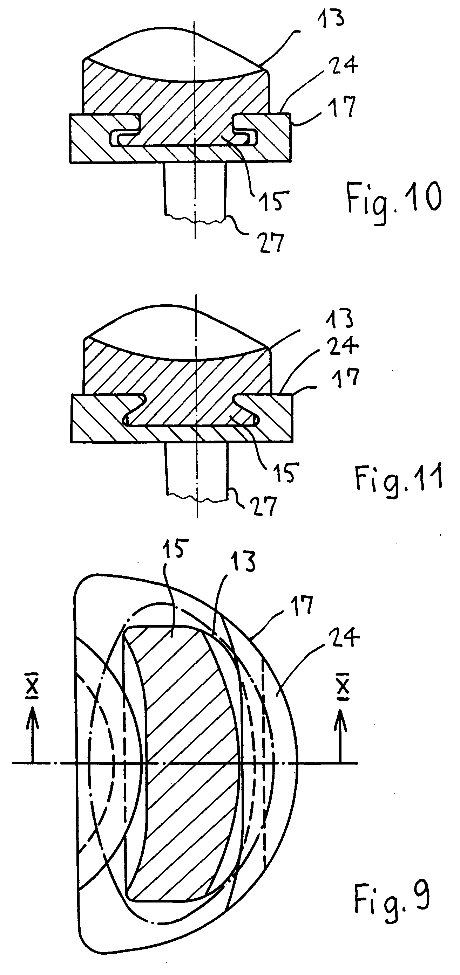

Beim Ausführungsbeispiel von Figur 5 ist der Radius R3 der äusseren Seitenwand 40 des Führungsorgans 15 etwa gleich gross, jedoch nicht grösser als der Radius R4 eines konzentrischen Kreises 37 (Fig. 7) um die innere Seitenwand 38 der Führung 35, welcher den geraden Abschnitt 36′′′ der äusseren Seitenwand 36 berührt und etwa 1,3 bis 2,6 mal grösser ist als der Radius R1 der Krümmung der inneren Seitenwand 38. Der Radius R3 kann jedoch kleiner gehalten sein, z.B. für eine elliptische Formgebung der äusseren Seitenwand 40. Der Radius R2 der Krümmung der äusseren Seitenwand 36 ist etwa gleich gross wie der Radius R3 der äusseren Seitenwand 40 des Führungsorgans 15. Es ist jedoch möglich, die Krümmung 36′, 36" flacher zu gestalten. Figur 8 zeigt auch die Radien R5 der Abschnitte 42′, 42′′ der Seitenwand 42. Der Radius R5 ist gleich gross oder etwas grösser als der Radius R1 (Fig. 7).

Die beschriebene Formgebung der Seitenwände von Führung 35 und Führungsorgan 15 ermöglicht es, das seitliche Spiel gering zu halten. Mit anderen Worten heisst dies, dass die Zwischenteile 13, gleich in welcher Lage sie sich befinden, in Richtung der Achse 44 nur um einen geringen Betrag verschoben werden können. Das Gelenk besitzt daher eine sehr hohe Querstabilität.

Beim Ausführungsbeispiel gemäss den Figuren 5 und 6 hat das Führungsorgan einen rechteckigen Querschnitt. Wie aber die Figuren 6C, 10 und 11 zeigen kann der Querschnitt z.B. auch T-oder schwalbenschwanzförmig sein. Durch eine solche Querschnittsform wird die Beweglichkeit der Zwischenteile 13 auf Bewegungen in der Ebene 24 begrenzt.

Bei einer Betrachtung der Figuren 5 und 6 kann man erkennen, dass die praktisch spiegelsymmetrische Anordnung von Führungen der beschriebenen Ausgestaltung den Zwischenteilen 13 erlaubt, sowohl eine Rotation um die Achse 39 als auch eine Translationsbewegung von anterior nach posterior und umgekehrt durchzuführen, wobei die Kongruenz zwischen den Kondylen 19 und den Lagerflächen 21 bestehen bleibt. Die genannten Bewegungen werden nach dem Einbau der Prothese durch die Bänder des Kniegelenks geführt und begrenzt. Der Bewegungsablauf bei Flexion und Extension entspricht weitgehend dem physiologischen Bewegungsablauf des Kniegelenks.

Die in der Zeichnung dargestellte Gestaltung von Führung 35 und Führungsorgan 15 erlaubt die Rotation um die Achse 39, ohne dass die verschleissmindernde Kongruenz der Kondylen 19 und der Lagerflächen gestört wird.

Das erste und das zweite Prothesenteil 11, 17 bestehen vorteilhaft aus einer Metallegierung, wie sie üblicherweise für Gelenkprothesen Anwendung findet. Die Zwischenteile 13 bestehen zweckmässigerweise aus einem Kunststoff, wie er ebenfalls üblicherweise für Gelenkprothesen verwendet wird.

Beim gezeigten Ausführungsbeispiel kommen zwei Kondylen/Lager flächen-Paare 19, 21 zur Anwendung. Es ist aber auch möglich, nur ein Kondylen/Lagerflächen-Paar zu verwenden. Denkbar wäre auch eine andere gelenkige Verbindung, z.B. ein durch einen Achsbolzen gekoppeltes Scharniergelenk, in Kombination mit der eine Translation und Rotation ermöglichenden Ausgestaltung von Zwischenteil 13 und Führungsorgan 15.

Bei der Variante gemäss den Figuren 5A, 6A und 6C ist die Prothese genau gleich ausgebildet wie in den Figuren 5 und 6, weist aber noch zusätzlich ein Verbindungsteil 49 auf, der die beiden Zwischenteile 13 starr miteinander koppelt. Dies verhindert eine Relativbewegung zwischen den Zwischenteilen 13 und damit eine Inkongruenz zwischen den Kondylen 19 und den Lagerflächen 21. Wie bereits einleitend erwähnt wurde, wird durch das Verbindungsteil 49 auch die Luxationssicherheit erhöht. Das Verbindungsteil 49 ist etwa brillenförmig und besteht vorteilhaft aus Metall oder faserverstärktem Kunststoff. Die Brillenöffnungen 51 sind der Kontur der Zwischenteile 13 angepasst. Das Verbindungsteil 49 kann daher nach dem Einsetzen der Zwischenteile 13 einfach über diese Zwischenteile gestülpt werden.

Eine andere Ausbildung des Verbindungsteils 49 ist aus den Figuren 5B und 6B ersichtlich. Hier ist das Verbindungsteil 49 eine U-förmige Klammer. In diesem Fall sind die Zwischenteile 13 mit Bohrungen 53 versehen, um die Arme 55 der Klammer 49 aufzunehmen. Die Klammer 49 wird nach dem Einsetzen der Zwischenteile 13 eingesetzt.

Beim gezeigten Ausführungsbeispiel von Fig. 6 und den Varianten gemäss den Figuren 6A und 6B sind die Kondylen 19 des ersten Prothesenteils 11 konvex und die Lagerflächen 21 der Zwischenteile 13 entsprechend konkav. Dies entspricht dem natürlichen Kniegelenk. Es wäre aber auch möglich, dort wo es die Form des Gelenks nahelegt, am ersten Prothesenteil statt Kondylen, konkave Lagerflächen und im Zwischenteil statt konkave Lagerflächen konvexe Kondylen vorzusehen.

Das Ausführungsbeispiel einer monokompartimentären Prothese gemäss den Figuren 9,10A und 10B entspricht praktisch einem Abschnitt der Prothese von Figur 5. Im übrigen ist jedoch die Ausgestaltung gleich, so dass auf die vorangehende Beschreibung verwiesen werden kann.

Wie in den Figuren 12, 13A und 13B dargestellt, ist es möglich, die Führung 35 im jeweiligen Zwischenstück 13 und das Führungsorgan 15 am zweiten Prothesenteil 17 auszubilden. Im übrigen entspricht dieses Ausführungsbeispiel jenen der Figuren 5 und 6, so dass für weitere Details auf die dortige Beschreibung verwiesen werden kann.

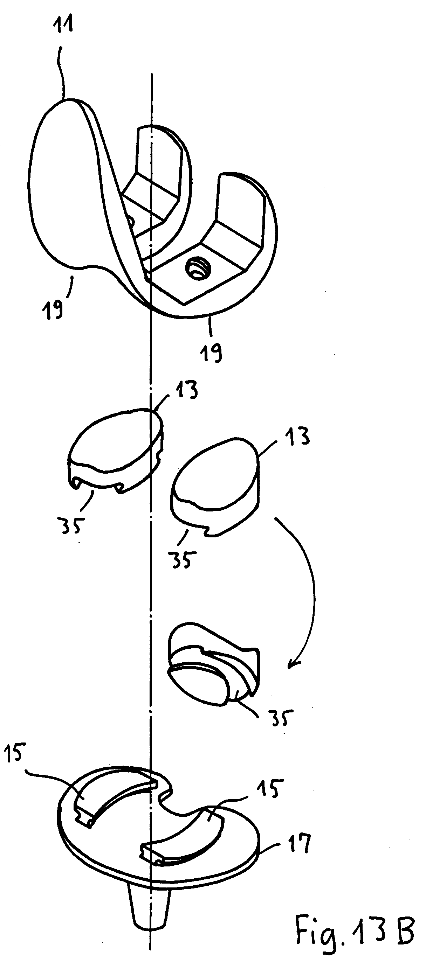

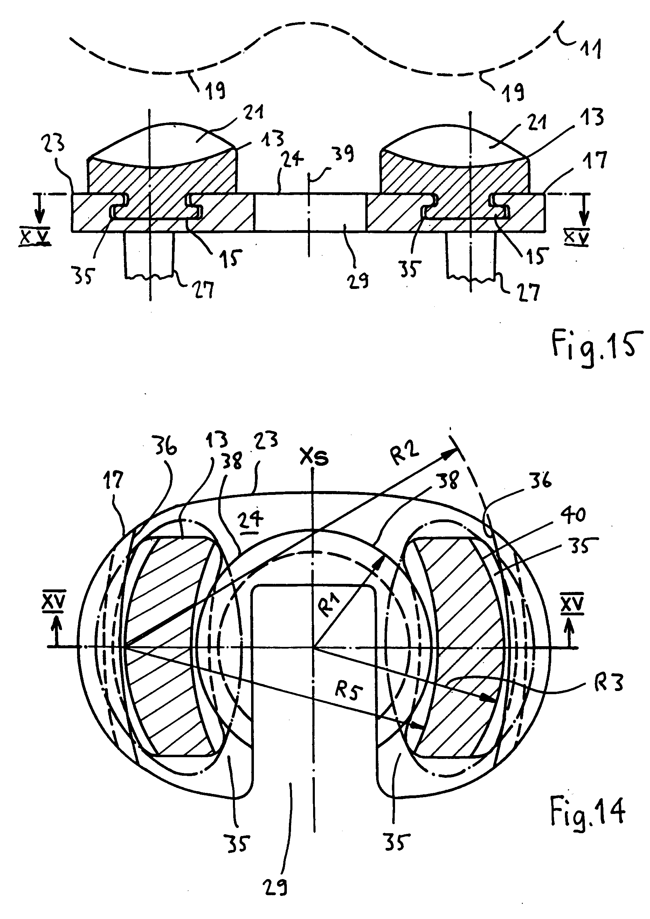

Bei der Ausführungsform von Figuren 14 und 15 ist der Aufbau der Prothese ähnlich wie bei jener gemäss den Figuren 5 und 6.

Das erste Prothesenteil 11 kann in herkömmlicher Weise mindestens einen Verankerungsabschnitt und mindestens einen Drehgelenkabschnitt aufweisen. Wiederum ist natürlich von Bedeutung, dass der Drehgelenkabschnitt des ersten Prothesenteils mit jenem des Zwischenteils übereinstimmt. Wie in Figur 15 dargestellt, wird der Drehgelenkabschnitt des ersten Prothesenteils 11 durch zwei Kondylen 19 gebildet. Diesen Kondylen 19 entsprechen die Lagerflächen 21 der Zwischenteile 13.

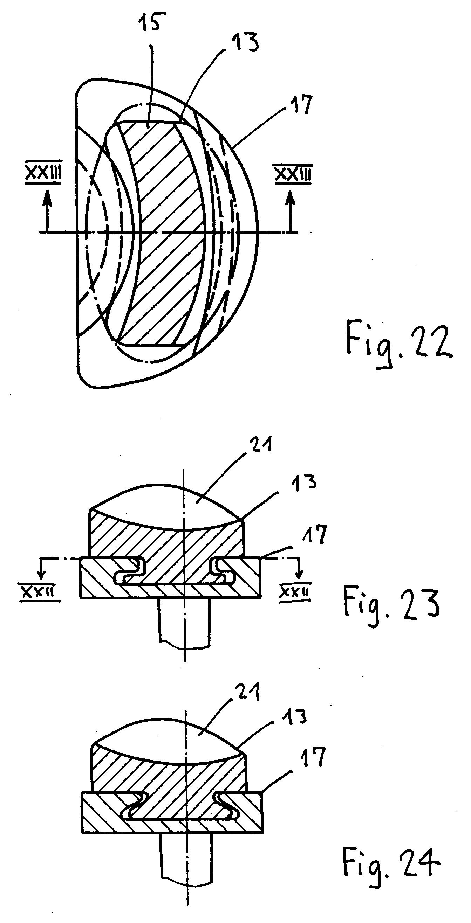

Das zweite Prothesenteil 17 besteht aus einer eine Gleitfläche 24 aufweisenden Platte 23, von welcher sich konische Verankerungsabschnitte 27 nach unten erstrecken. Wie die Figuren 14 und 21 zeigen, besitzt die Platte 23 eine den Umrissen der natürlichen Tibiakondylen angepasste ovale Formgebung mit einer posterioren Ausnehmung 29 für mindestens das hintere Kreuzband (Fig. 21). Es ist jedoch möglich und ein Vorteil der vorliegenden Erfindung, dass die Ausnehmung 29 so tief gestaltet werden kann, dass sie auch das vordere Kreuzband umfasst (Fig. 14). Im Prothesenteil 17 befinden sich zwei Führungsbahnen 35, welche die Form einer T- oder Schwalbenschwanz-Nut aufweisen. Sie könnten aber auch einen anderen geeigneten Querschnitt aufweisen. Jede Nut 35 ist gegen anterior oder posterior aufgeweitet, wobei die Seitenwände 36, 38 der Nut 35 eine Krümmung aufweisen. Der Abstand zwischen den Seitenwänden 36, 38 ist etwa in der Mitte der Führung am kleinsten. Beim Ausführungsbeispiel von Fig. 14 ist der Radius R2 der Krümmung der äusseren Seitenwand 36 etwa dreimal grösser als der Radius R1 der Krümmung der inneren Seitenwand 38.

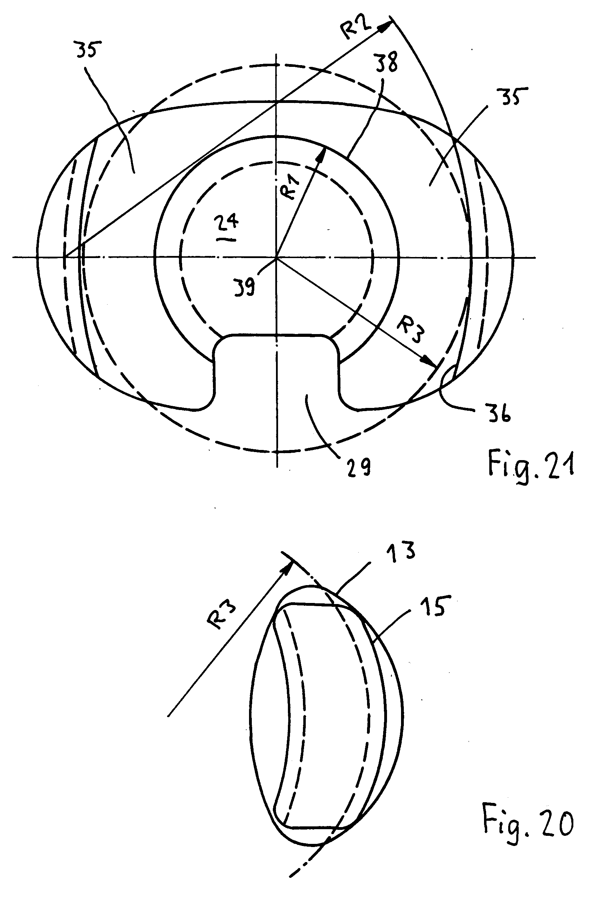

Die Formgebung der Zwischenteile 13 ist insbesondere aus den Figuren 14, 15 und 20 ersichtlich. Die Zwischenteile besitzen oben eine Lagerfläche 21 und unten ein der Form der Nut 35 angepasstes Führungsorgan 15. Das Führungsorgan 15 hat also eine längliche gekrümmte Form und ist im Querschnitt z.B. T-oder schwalbenschwanzförmig. Durch eine solche Querschnittsform wird die Beweglichkeit der Zwischenteile 13 auf Bewegungen in der Ebene 24 begrenzt.

Der Radius der Kurve R3 der äusseren Wandung 40 des Führungsorgans 15 ist kleiner als der Radius R5 der inneren Wandung 42. Deshalb hat das Führungsorgan die grösste Breite etwa in der Mitte.

Fig. 14 zeigt auch das Spiel zwischen dem Führungsorgan 15 und der Führungsbahn 35. Das Spiel beträgt an der engsten Stelle insgesamt etwa 0,5 bis 3 mm, wenn sich das Zwischenteil 13 in der eingezeichneten Mittellage befindet.

Der Radius R3 entspricht vorteilhaft der Summe von R1 und dem inneren Spiel di und der grössten Breite wo des Führungsorgans, und der Radius R5 der Differenz zwischen dem Radius R2 und Summe der grössten Breite wo und dem äusseren Spiel wo.

Bei einer Betrachtung der Figuren 14 und 15 kann man erkennen, dass die praktisch spiegelsymmetrische Anordnung von Führungen der beschriebenen Ausgestaltung den Zwischenteilen 13 erlaubt, sowohl eine Rotation um die Achse 39 als auch eine Translationsbewegung von anterior nach posterior und umgekehrt durchzuführen, wobei die Kongruenz zwischen den Kondylen 19 und den Lagerflächen 21 bestehen bleibt. Die genannten Bewegungen werden nach dem Einbau der Prothese durch die Bänder des Kniegelenks geführt und begrenzt. Der Bewegungsablauf bei Flexion und Extension entspricht weitgehend dem physiologischen Bewegungsablauf des Kniegelenks.

Die in der Zeichnung dargestellte Gestaltung von Führung 35 und Führungsorgan 15 erlaubt die Rotation um die Achse 39, ohne dass die verschleissmindernde Kongruenz der Kondylen 19 und der Lagerflächen gestört wird.

Das erste und das zweite Prothesenteil 11, 17 bestehen vorteilhaft aus einer Metallegierung, wie sie üblicherweise für Gelenkprothesen Anwendung findet. Die Zwischenteile 13 bestehen zweckmässigerweise aus einem Kunststoff, wie er ebenfalls üblicherweise für Gelenkprothesen verwendet wird.

Beim gezeigten Ausführungsbeispiel kommen zwei Kondylen/Lagerflächen-Paare 19, 21 zur Anwendung. Es ist aber auch möglich, nur ein Kondylen/Lagerflächen-Paar zu verwenden. Denkbar wäre auch eine andere gelenkige Verbindung, z.B. ein durch einen Achsbolzen gekoppeltes Scharniergelenk, in Kombination mit der eine Translation und Rotation ermöglichenden Ausgestaltung von Zwischenteil 13 und Führungsorgan 15.

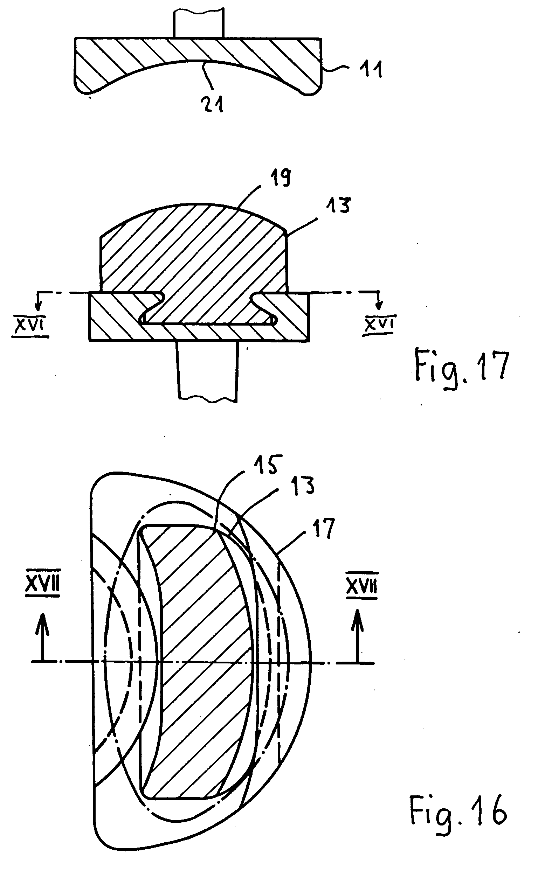

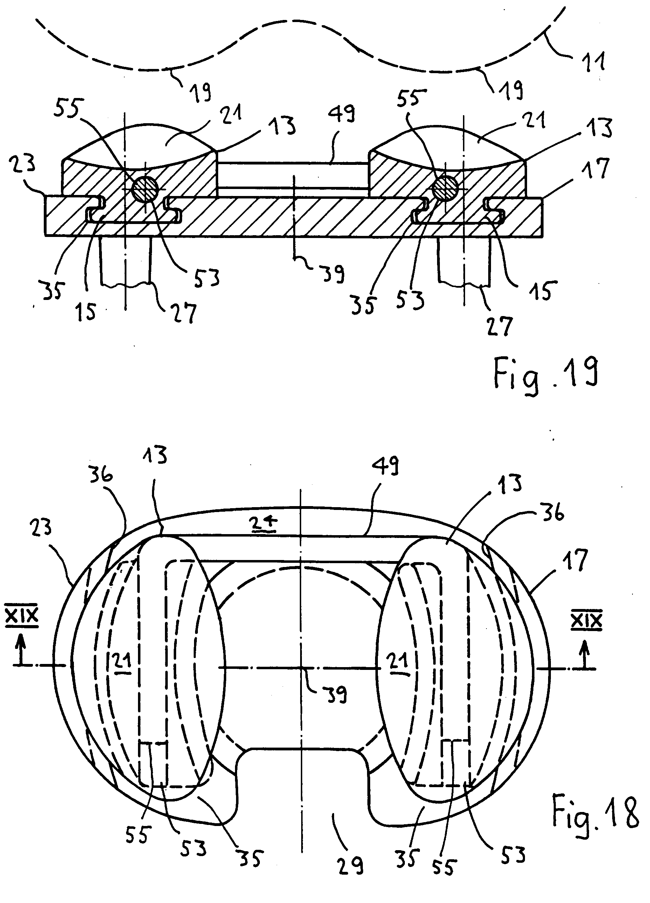

Die Figuren 16 und 17 zeigen eine Anordnung, welche ähnlich ist, wie die Anordnung in den Figuren 9 und 11, wobei jedoch das Zwischenteil 13 eine Kondyle 19 aufweist und der erste Prothesenteil 11 eine konkave Lagerfläche 21.

Bei der Variante gemäss den Figuren 18 bis 21 ist die Prothese gleich ausgebildet wie in den Figuren 14 und 15, weist aber noch zusätzlich ein Verbindungsteil 49 auf, der die beiden Zwischenteile 13 starr miteinander koppelt. Dies verhindert eine Relativbewegung zwischen den Zwischenteilen 13 und damit eine Inkongruenz zwischen den Kondylen 19 und den Lagerflächen 21. Wie bereits einleitend erwähnt wurde, wird durch den Verbindungsteil 49 auch die Luxationssicherheit erhöht.

Das Verbindungsteil 49 ist eine U-förmige Klammer. Die Zwischenteile 13 sind mit Bohrungen 53 versehen, um die Arme 55 der Klammer 49 aufzunehmen. Die Klammer 49 wird nach dem Einsetzen der Zwischenteile 13 eingesetzt.

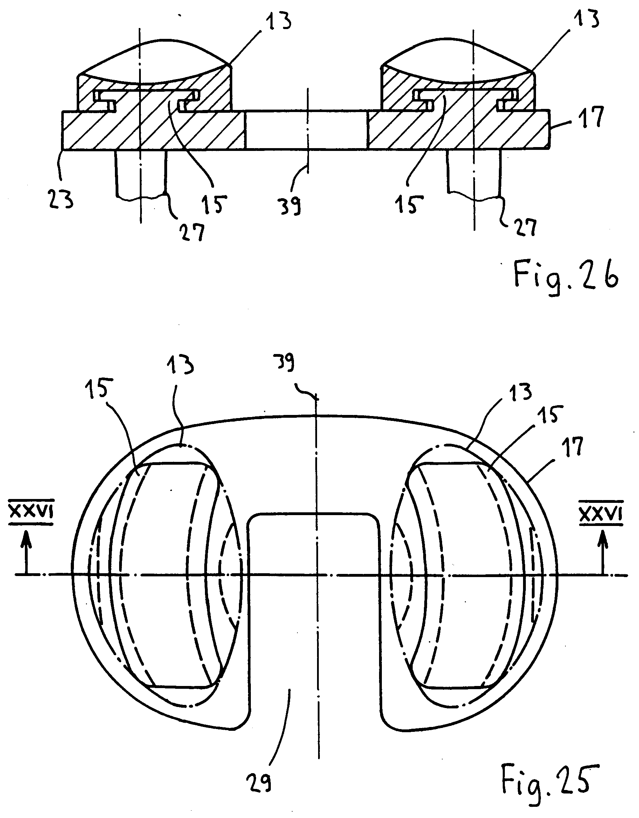

Das Ausführungsbeispiel einer monokompartimentären Prothese gemäss den Figuren 22 und 23 entspricht praktisch einem Abschnitt der Prothese von Figur 14 und 15. Im übrigen ist jedoch die Ausgestaltung gleich, so dass auf die vorangehende Beschreibung verwiesen werden kann.

Wie in den Figuren 25 und 26 dargestellt, ist es möglich, die Führung 35 im jeweiligen Zwischenstück 13 und das Führungsorgan 15 am zweiten Prothesenteil 17 auszubilden. Im übrigen entspricht dieses Ausführungsbeispiel jenen der Figuren 5 und 6, so dass für weitere Details auf die dortige Beschreibung verwiesen werden kann.

Es sind weiter noch viele Aenderungen möglich, ohne vom Erfindungsgedanken abzuweichen. So können verschiedene Merkmale, die in den einzelnen Figuren dargestellt sind, kombiniert werden. So kann beispielsweise die Gelenkprothese von Figur 26 auch eine Schwalbenschwanzführung aufweisen.

## Patentansprüche

1. Gelenkprothese, insbesondere Kniegelenkprothese, mit mindestens einem ersten Prothesenteil (11), das einen Verankerungsabschnitt und mindestens einen Gelenkabschnitt (19) aufweist, mindestens einem zweiten Prothesenteil (17), das einen Verankerungsabschnitt (27) und eine Platte (23) aufweist, und mindestens einem Zwischenteil (13), welches mit dem Gelenkabschnitt des ersten Prothesenteils eine Artikulation ermöglicht und durch Zusammenwirken einer Führung (35) mit einem in die Führung eingreifenden Führungsorgan (15) geführt von anterior nach posterior und umgekehrt auf der Platte (23) des zweiten Prothesenteils (17) verschiebbar ist, dadurch gekennzeichnet, dass die Führung (35) gegen anterior und posterior aufgeweitet ist, um dem Zwischenteil (13) eine entsprechende Bewegungsfreiheit zu gestatten.

2. Gelenkprothese nach Anspruch 1, dadurch gekennzeichnet, dass die jeweilige Führung durch eine Seitenwände (36, 38) aufweisende Nut (35) in der Platte (23) oder im Zwischenteil (13) gebildet ist.

3. Gelenkprothese nach Anspruch 2, dadurch gekennzeichnet, dass die Seitenwände (36, 38) der Nut (35) eine Krümmung aufweisen und dass das Führungsorgan (15) eine länglich gekrümmte Form aufweist.

4. Gelenkprothese nach Anspruch 2 oder 3, dadurch gekennzeichnet, dass die äussere Seitenwand (36) der Nut (35) zumindest in der Mitte einen praktisch geraden Abschnitt (36′′′) aufweist und dass die innere Seitenwand (42) des Führungsorgans (15) in der Mitte einen praktisch geraden Abschnitt (42′′′) aufweist.

5. Gelenkprothese nach einem der Ansprüche 1 bis 4, dadurch gekennzeichnet, dass der Abstand zwischen den Seitenwänden (36, 38) der Nut (35) etwa in der Mitte der Bahnlänge am kleinsten ist und dass die Breite des Führungsorgans (15) über dessen Länge nicht ganz diesem Abstand entspricht.

6. Gelenkprothese nach einem der Ansprüche 3 bis 5, dadurch gekennzeichnet, dass der Radius (R3) der Krümmung der äusseren Seitenwand (40) des Führungsorgans (15) gleich gross oder kleiner ist als der Radius (R4) eines konzentrischen Kreises (37) um die innere Seitenwand (38) der Führung (35), welcher den geraden Abschnitt (36′′′) der äusseren Seitenwand (36) der Führung (35) berührt.

7. Gelenkprothese nach Anspruch 6, dadurch gekennzeichnet, dass der Radius (R4) des konzentrischen Kreises (37) etwa 1,3 bis 2,6 mal grösser ist als der Radius (R1) der Krümmung der inneren Seitenwand (38) der Führung (35).

8. Gelenkprothese nach einem der Ansprüche 1 bis 7, dadurch gekennzeichnet, dass das seitliche Spiel in der Führung (35) an deren engster Stelle des Führungsorgans (15) 0,5 bis 3 mm beträgt.

9. Gelenkprothese nach einem der Ansprüche 3 bis 8, dadurch gekennzeichnet, dass der Radius (R2) der Krümmung der äusseren Seitenwand (36) der Nut (35) etwa dreimal grösser ist als der Radius (R1) der Krümmung der inneren Seitenwand (38).

10. Gelenkprothese nach einem der Ansprüche 3 bis 9, dadurch gekennzeichnet, dass der Radius (R3) der Krümmung der äusseren Seitenwand (40) des Führungsorgans (15) etwa halb so gross ist wie der Radius (R2) der Krümmung der äusseren Seitenwand der Nut (35).

11. Gelenkprothese nach einem der Ansprüche 1 bis 10, dadurch gekennzeichnet, dass die Beweglichkeit des Zwischenteils (13) relativ zur Führung (35) auf Bewegungen in der Ebene (24) eingeschränkt ist.

12. Gelenkprothese nach einem der Ansprüche 1 bis 10, dadurch gekennzeichnet, dass die Nut (35) und das Führungsorgan (15) einen T-förmigen Querschnitt aufweisen.

13. Gelenkprothese nach einem der Ansprüche 1 bis 10, dadurch gekennzeichnet, dass die Nut (35) und das Führungsorgan (15) einen schwalbenschwanzförmigen Querschnitt aufweisen.

14. Gelenkprothese nach einem der Ansprüche 1 bis 13, dadurch gekennzeichnet, dass zwei Kondylen (19) vorgesehen sind und dass für jede Kondyle (19) ein Zwischenteil (13) mit einer entsprechenden Lagerfläche (21) für die Kondyle (19) vorgesehen ist.

15. Gelenkprothese nach Anspruch 14, dadurch gekennzeichnet, dass die Führungen (35) so angeordnet sind, dass sie anterior und posterior konvergieren.

16. Gelenkprothese nach Anspruch 14 oder 15, dadurch gekennzeichnet, dass ein Verbindungsteil (49) vorgesehen ist, welcher die beiden Zwischenteile (13) miteinander koppelt.

17. Gelenkprothese nach Anspruch 16, dadurch gekennzeichnet, dass das Verbindungsteil (49) brillenförmig ist und über die Zwischenteile (13) gestülpt ist.

18. Gelenkprothese nach Anspruch 17, dadurch gekennzeichnet, dass das Verbindungsteil (49) eine U-förmige Klammer ist und dass die Zwischenteile Oeffnungen (53) zum Einstecken der Klammer aufweisen.

19. Gelenkprothese nach einem der Ansprüche 1 bis 13, dadurch gekennzeichnet, dass der Drehgelenkabschnitt des ersten Prothesenteils (11) durch eine Kondyle (19) gebildet ist und dass für diese Kondyle (19) eine Lagerfläche (21) auf einem Zwischenteil (13) vorgesehen ist.

20. Gelenkprothese nach einem der Ansprüche 1 bis 19, dadurch gekennzeichnet, dass die jeweilige Kondyle (19) des ersten Prothesenteils (11) konvex und die zugehörige Lagerfläche (21) des Zwischenteils (13) entsprechend konkav ist.

21. Gelenkprothese nach einem der Ansprüche 1 bis 20, dadurch gekennzeichnet, dass die jeweilige Kondyle (19) des ersten Prothesenteils (11) konkav und die entsprechende Lagerfläche (21) des Zwischenteils (13) konvex ist.

22. Gelenkprothese nach einem der Ansprüche 1 bis 21, dadurch gekennzeichnet, dass das zweite Prothesenteil (17) und das Zwischenteil (13) eine Ausnehmung (29) für das vordere und/oder hintere Kreuzband aufweist.

## Claims

1. A joint prosthesis, more particularly a knee-joint prosthesis, comprising at least one first part (11) comprising an anchoring portion and at least one joint portion (19), at least one second part (17) comprising an anchoring portion (27) and a plate (23), and at least one intermediate part (13) which provides an articulation with the joint portion of the first part and, in co-operation with a guide (35) and a guide means (15) engaging in the guide, is guided from the anterior to the posterior direction and vice versa and movable on the plate (23) of the second prosthesis part (17), characterised in that the guide (35) is widened in the anterior and posterior direction, to provide appropriate freedom of motion to the intermediate part (13).

2. A joint prosthesis according to claim 1, characterised in that in each case the guide is formed by a groove (35) with side walls (36, 38) in the plate (23) or in the intermediate part (13).

3. A joint prosthesis according to claim 2, characterised in that the side walls (36, 38) of the groove (35) are curved and the guide means (15) has a longitudinally curved shape.

4. A joint prosthesis according to claim 2 or 3, characterised in that the outer side wall (36) of the groove (35) has a substantially straight portion (36''') at least in the middle, and the inner side wall (42) of the guide means (15) has a practically straight portion (42''') in the middle.

5. A joint prosthesis according to any of claims 1 to 4, characterised in that the distance between the side walls (36, 38) of the groove (35) is smallest approximately in the middle of the track length, and the width of the guide means (15) along this length is not quite equal to the aforementioned distance.

6. A joint prosthesis according to any of claims 3 to 5, characterised in that the radius (R3) of curvature of the outer side wall (40) of the guide means (15) is equal to or less than the radius (R4) of a concentric circle (37) around the inner side wall (38) of the guide (35) and touching the straight portion (36''') of the outer side wall (36) of the guide (35).

7. A joint prosthesis according to claim 6, characterised in that the radius (R4) of the concentric circle (37) is about 1.3 to 2.6 times as great as the radius (R1) of curvature of the inner side wall (38) of the guide (35).

8. A joint prosthesis according to any of claims 1 to 7, characterised in that the lateral clearance in the guide (35) at the narrowest point of the guide means (15) is 0.5 to 3 mm.

9. A joint prosthesis according to any of claims 3 to 8, characterised in that the radius (R2) of curvature of the outer side wall (36) of the groove (35) is about three times the radius (R1) of curvature of the inner side wall (38).

10. A joint prosthesis according to any of claims 3 to 9, characterised in that the radius (R3) of curvature of the outer side wall (40) of the guide means (15) is about half the radius (R2) of curvature of the outer side wall of the groove (35).

11. A joint prosthesis according to any of claims 1 to 10, characterised in that the mobility of the intermediate part (13) relative to the guide (35) is restricted to motion in the plane (24).

12. A joint prosthesis according to any of claims 1 to 10, characterised in that the groove (35) and the guide means (15) have a T-shaped cross-section.

13. A joint prosthesis according to any of claims 1 to 10, characterised in that the groove (35) and the guide means (15) have a dovetail cross-section.

14. A joint prosthesis according to any of claims 1 to 13, characterised in that two condyles (19) are provided and an intermediate part (13) with a corresponding bearing surface (21) is provided for each condyle (19).

15. A joint prosthesis according to claim 14, characterised in that the guides (35) are disposed so that they converge in the anterior and posterior direction.

16. A joint prosthesis according to claim 14 or 15, characterised in that a connecting part (49) is provided and couples the two intermediate parts (13).

17. A joint prosthesis accordion to claim 16, characterised in that the connecting part (49) is spectacle-shaped and placed over the intermediate parts (13).

18. A joint prosthesis according to claim 17, characterised in that the connecting part (49) is a U-shaped bracket and the intermediate parts have openings (53) for inserting the bracket.

19. A joint prosthesis according to any of claims 1 to 13, characterised in that the pivot-joint portion of the first prosthesis part (11) is formed by a condyle (19), and the condyle (19) is provided with a bearing surface (21) on an intermediate part (13).

20. A joint prosthesis according to any of claims 1 to 19, characterised in that the condyle (19) on the first prosthesis part (11) is convex and the associated bearing surface (21) on the intermediate part (13) is correspondingly concave.

21. A joint prosthesis according to any of claims 1 to 20, characterised in that the condyle (19) on the first prosthesis part (11) is concave and the corresponding bearing surface (21) on the intermediate part (13) is convex.

22. A joint prosthesis according to any of claims 1 to 21, characterised in that the second prosthesis part (17) and the intermediate part (13) has a recess (29) for the front and/or rear cruciate ligament.

## Revendications

1. Prothèse articulaire, en particulier prothèse du genou, comprenant au mains une première partie de prothèse (11) qui présente un segment d'ancrage et au moins un segment articulaire (19), au moins une seconde partie de prothèse (17) qui présente un segment d'ancrage (27) et un plateau (23), et au moins une partie intermédiaire (13) qui rend possible une articulation avec le segment articulaire de la première partie de prothèse et peut subir un déplacement guide d'avant en arrière et inversement sur le plateau (23) de la seconde partie de prothèse (17) par coopération d'un guide (35) avec un organe de guidage (15) en prise avec le guide, caractérisée en ce que le guide (35) est élargi vers l'avant et vers l'arrière afin de donner à la partie intermédiaire (13) une liberté de déplacement convenable.

2. Prothèse articulaire selon la revendication 1, caractérisée en ce que le guide est constitué par une rainure (35) comportant des parois latérales (36, 38) dans le plateau (23) ou dans la partie intermédiaire (13).

3. Prothèse articulaire selon la revendication 2, caractérisée en ce que les parois latérales (36, 38) de la rainure (35) présentent une courbure et en ce que l'organe de guidage (15) présente une forme courbe allongée.

4. Prothèse articulaire selon la revendication 2 ou 3, caractérisée en ce que la paroi latérale externe (36) de la rainure (35) présente, au moins au milieu, un segment pratiquement rectiligne (36''') et en ce que la paroi latérale interne (42) de l'organe de guidage (15) présente au milieu un segment pratiquement rectiligne (42''').

5. Prothèse articulaire selon l'une quelconque des revendications 1 à 4, caractérisée en ce que la distance entre les parois latérales (36, 38) de la rainure (35) est minimale à peu près au milieu de la longueur de la trajectoire et en ce que la largeur de l'organe de guidage (15) ne correspond pas complètement à cette distance sur toute sa longueur.

6. Prothèse articulaire selon l'une quelconque des revendications 3 à 5, caractérisée en ce que le rayon (R3) de la courbure de la paroi latérale externe (40) de l'organe de guidage (15) est égal ou inférieur au rayon (R4) d'un cercle concentrique (37) autour de la paroi latérale interne (38) du guide (35), tangent au segment rectiligne (36''') de la paroi latérale externe (36) du guide (35).

7. Prothèse articulaire selon la revendication 6, caractérisée en ce que le rayon (R4) du cercle concentrique (37) est à peu près 1,3 à 2,6 fois plus grand que le rayon (R1) de la courbure de la paroi latérale interne (38) du guide (35).

8. Prothèse articulaire selon l'une quelconque des revendications 1 à 7, caractérisée en ce que le jeu latéral de l'organe de guidage (15) dans le guide (35) au point le plus étroit de celui-ci est de 0,5 à 3 mm.

9. Prothèse articulaire selon l'une quelconque des revendications 3 à 8, caractérisée en ce que le rayon (R2) de la courbure de la paroi latérale externe (36) de la rainure (35) est à peu près trois fois plus grand que le rayon (R1) de la courbure de la paroi latérale interne (38).

10. Prothèse articulaire selon l'une quelconque des revendications 3 à 9, caractérisée en ce que le rayon (R3) de la courbure de la paroi latérale externe (40) de l'organe de guidage (15) est à peu près deux fois moins grand que le rayon (R2) de la courbure de la paroi latérale externe de la rainure (35).

11. Prothèse articulaire selon l'une quelconque des revendications 1 à 10, caractérisée en ce que la mobilité de la partie intermédiaire (13) par rapport au guide (35) est limitée à des mouvements dans le plan (24).

12. Prothèse articulaire selon l'une quelconque des revendications 1 à 10, caractérisée en ce que la rainure (35) et l'organe de guidage (15) présentent une section transversale en forme de T.

13. Prothèse articulaire selon l'une quelconque des revendications 1 à 10, caractérisée en ce que la rainure (35) et l'organe de guidage (15) présentent une section transversale en queue d'aronde.

14. Prothèse articulaire selon l'une quelconque des revendications 1 à 13, caractérisée en ce qu'il est prévu deux condyles (19) et en ce qu'il est prévu, pour chaque condyle (19), une partie intermédiaire (13) comportant une surface portante correspondante (21) pour le condyle (19).

15. Prothèse articulaire selon la revendication 14, caractérisée en ce que les guides (35) sont disposés de sorte qu'ils convergent en avant et en arrière.

16. Prothèse articulaire selon la revendication 14 ou 15, caractérisée en ce qu'il est prévu une pièce de jonction (49) qui relie entre elles les deux parties intermédiaires (13).

17. Prothèse articulaire selon la revendication 16, caractérisée en ce que la pièce de jonction (49) est en forme de lunettes et est rabattue sur les parties intermédiaires (13).

18. Prothèse articulaire selon la revendication 17, caractérisée en ce que la pièce de jonction (49) est un cavalier de jonction en U et en ce que les parties intermédiaires présentent des ouvertures (53) pour l'insertion du cavalier de jonction.

19. Prothèse articulaire selon l'une quelconque des revendications 1 à 13, caractérisée en ce que le segment articulaire rotatif de la première partie de prothèse (11) est constitué par un condyle (19) et en ce qu'il est prévu, pour ce condyle (19), une surface portante (21) sur une partie intermédiaire (13).

20. Prothèse articulaire selon l'une quelconque des revendications 1 à 19, caractérisée en ce que le condyle (19) de la première partie de prothèse (11) est convexe et la surface portante associée (21) de la partie intermédiaire (13) présente une concavité correspondante.

21. Prothèse articulaire selon l'une quelconque des revendications 1 à 20, caractérisée en ce que le condyle (19) de la première partie de prothèse (11) est concave et la surface portante correspondante (21) de la partie intermédiaire (13) est convexe.

22. Prothèse articulaire selon l'une quelconque des revendications 1 à 21, caractérisée en ce que la seconde partie de prothèse (17) et la partie intermédiaire (13) présentent un creux (29) pour le ligament cruciforme antérieur et/ou postérieur.
